# EUROPEAN PATENT SPECIFICATION

(11) **EP 2 433 501 B1**
(45) Date of publication and mention of the grant of the patent: **12.07.2017**
(21) Application number: 11195342.8
(22) Date of filing: 12.09.2008
(51) Int. Cl.: A23K 10/30, A23K 10/12, A23K 50/75, A23L 19/00, A23L 33/105

(54) **AGENT FOR INCREASING BODY WEIGHT, ANIMAL FEED FOR INCREASING BODY WEIGHT AND HEALTH FOOD FOR INCREASING BODY WEIGHT**
MITTEL ZUM ERHÖHEN DES KÖRPERGEWICHTS, TIERFUTTER ZUM ERHÖHEN DES KÖRPERGEWICHTS UND HEALTH FOOD ZUM ERHÖHEN DES KÖRPERGEWICHTS
AGENT DE PRISE DE POIDS, ALIMENT FAVORISANT LA PRISE DE POIDS CHEZ L'ANIMAL ET ALIMENT DIÉTÉTIQUE DESTINÉ À FAVORISER LA PRISE DE POIDS CHEZ L'ÊTRE HUMAIN

(30) Priority: 12.10.2007 JP 2007266156
(43) Date of publication of application: 28.03.2012
(62) Divisional of application: 08837559.7
(73) Proprietor: Kraft Co., Ltd., Kagawa 760-0017 (JP); National University Corporation Kagawa University, Takamatsu-shi, Kagawa 760-8521 (JP); National University Corporation Kochi University, Kochi-shi, Kochi 780-8520 (JP); Rels Co., Ltd., Befu-cho Kakogawa-shi Hyogo 675-0131 (JP); Ota, Hiroto, Mure-cho Takamatsu-shi Kagawa 761-0121 (JP)
(72) Inventor: Hasui, Kazumori, Takamatsu-shi, Kagawa 760-0017 (JP); Hasui, Yuta, Takamatsu-shi, Kagawa 760-0017 (JP); Yamauchi, Koh-en, Kita-gun, Kagawa 761-0795 (JP); Fukada, Haruhisa, Nankoku-shi, Kochi 783-8502 (JP)
(74) Representative: Wilson Gunn

(56) References cited:
- KIM J H ET AL: "Effect of Mugwort processing types on in vivo digestibility and ruminal fermentation characteristics in sheep", JOURNAL OF ANIMAL SCIENCE AND TECHNOLOGY, vol. 47, no. 3, June 2005 (2005-06), pages 409-418, XP002669249, ISSN: 1598-9429
- DATABASE WPI Week 200340 Thomson Scientific, London, GB; AN 2003-424005 XP002669250, & JP 2002 330725 A (RYUKYU BIORESOURCE KAIHATSU KK) 19 November 2002 (2002-11-19)
- DATABASE WPI Week 200701 Thomson Scientific, London, GB; AN 2007-003508 XP002669251, & JP 2006 296245 A (KUBOI K) 2 November 2006 (2006-11-02)
- CANIBE ET AL: "Fermented liquid feed and fermented grain to piglets- effect on gastrointestinal ecology and growth performance", LIVESTOCK SCIENCE, ELSEVIER, AMSTERDAM, NL, vol. 108, no. 1-3, 25 June 2007 (2007-06-25), pages 198-201, XP022052827, ISSN: 1871-1413, DOI: 10.1016/J.LIVSCI.2007.01.095
- Mongkol Samanya ET AL: "Histological alterations of intestinal villi in chickens fed dried Bacillus subtilis var. natto", Comparative Biochemistry and Physiology Part A: Molecular & Integrative Physiology, vol. 133, no. 1, 1 September 2002 (2002-09-01), pages 95-104, XP055126950, ISSN: 1095-6433, DOI: 10.1016/S1095-6433(02)00121-6

## Description

### TECHNICAL FIELD

The present invention relates to a body weight gain agent, an animal body weight gain feed and a human body weight gain health food.

### TECHNICAL BACKGROUND

The inventor of the present invention has paid attention to that Zingiberis-Rhizoma-crudus, hence called as "ginger", is effective for deodorization of chicken-meat. The reason of it is that a chicken-meat which may smell and taste of ginger is expected. Then, the inventor of the present invention tried to give ginger powder to chicken by adding it to a basal feed. However, the experiment ended in complete failure. The reason for this is that, in the case of that an amount of addition of ginger is more, chicken does not voluntarily eat the feed. On the other hand, in the case of that an amount of addition of ginger is fewer, a chicken which smells and tastes of ginger can not obtained.

In the prior art, it is supposed that a herbal medicine which is effective for humans is also effective for livestock and poultry.
It is has been disclosed in Japanese Patent Laid Open No.01-172341 (Japanese Patent No.2599161) to add to a feed the herbal medicine selected from the group consisting of Glycyrrhizae-Radix, Artemisia-princeps, Coptis-japonica, Scutellariae-Radix, Phellodendri-Cortex, Geraniua-nepalense-Sweet, Magnoliae-Cortex, Salvia-alltlorrhiza-Bunge, Anemarrhenae-Rhizoma, Rhei-Rhizoma, Caryophylli Flos, Ligustrumjaponicum-Thunberg, Eriobotryae-Folium, Saposhnikoviaseseloides, Humulus-lupulus, Perillae-Herba, Schizonepetae-Spica, Cinnamomi-Cortex, Scrophularia-Oldhami-Oliver, Cassiae-Semen, Myricarubra-Sicbold-et-Zuccarini, Forsythiae-Fructus, Aloe, Bostaurusdomesticus-Gmelin, Clematidis-Radix, Prunus-Mume-Sieboldet-Zuccarini, Isodon-trichocarpus-Kudo, Aquilariaagallocha-Roxb., Cnidii-Rhizoma, Nothosmyrnium-japoniccum-Miquel, Isodon-trichocarpus-Kudo, Plantage-asiatica-Linnee, Magnoliae-Flos, Artemisiae-Capillari-Flos, Corni-Fructus Lithospermi-Radix Picrorrhiza-Kurooa-Royle-ex-Benth, Paeoniae-Radix, Rosa-Laevigata-Michaux, Thyme, Nandinadomestica-Thunb.-var.-leucocarpa-Makino, Sanguisorbaofficinalis-L., Ephedrae Herba.

It is supposed that a spice which is useful for humans is also effective for livestock and poultry. It is has been disclosed in Japanese Patent Laid Open No.01-075426 (Japanese Patent No.2535555) that the herbal medicine selected from the group consisting of Rhei-Rhizoma, Coptis-japonica, Phellodendri-Cortex, Magnoliae-Cortex, Artemisia-princeps, Geraniua-nepalense-Sweet, Sophorae-Radix, Isodon-trichocarpus-Kudo, Humulus-lupulus, Bostaurusdomesticus-Gmelin, Salvia-alltlorrhiza-Bunge, Glycyrrhizae-Radix is effective for fish. So that, it is also known to add these herbal medicines to the feed.

It is has been disclosed in Japanese Patent Laid Open No.2003-299445 (Japanese Patent No.3926193) to add to a feed the spice selected from the group consisting of Red-pepper, Clove, Ginger, Oregano, Nutmeg, Cinnamon, Pepper, Garlic, Onion, Sage, Rosemary, Maes, Allspice, Taragon, Parsley, Fenugreek, Red-cherry, Thyme, Star-anise, Mustard, Globe-artichoke, Chamomile, Chrysanthemum, Sunflower, Dandelion, Chicory, Yarrow, Laurel, Sesame, Catnip, Savory, Palm, Basil, Peppermint, Marjoram, Mint, Lavender, Peppermint, Savory, Galangal, Cardamon, Amomi-Semen, Tarmelic, Anise-hyssop, Caraway, Cumin, Coriander, Celery, Dill, Fennel, Paprika, Agrimony, Eucalyptus, Zanthoxyli-Fructus for retaining the freshness of sea-foods.

It is has been disclosed in Japanese Patent Laid Open No.10-23431 to add to a feed 0.05 % ∼0.5 % by weight of ginger for deodorization of pig excreta.

It is has been disclosed in Japanese Patent Laid Open No.07-079709 (Japanese Patent No.2917818) to add to a feed the spice selected from the group consisting of Nutmeg, Mace, Mustard, Zanthoxyli-Fructus, Taragon, Sesame, Black-pepper, White-pepper, Fenugreek, Caraway, Coriander, Cumin, Fennel, Parsley, Paprika, Clove, Allspice, Reddust, Marjoram, Rosemary, Oregano, Sage, Thyme, Laurel, Basil, Savory, Cinnamon, Tarmelic, Cardamon, Ginger for retaining the freshness of meat of livestock and poultry.

It is has been disclosed in Japanese Patent Laid Open No.2006-298871 that a plant extract fermented liquid, which has an excellent health effect, a good palatability, a good storability, and specially a good blood cholesterol concentration-lowering activity, is obtained by fermenting with at least a kind of bacteria-strain selected from Yeast and Lactic-acid-bacteria an extract which is extracted two or more raw materials which are selected from the group consisting of Apple, Carrot, Daikonradish, Cabbage, Celery, Cucumber, Banana, Onion, Edible-burdock, Spinach, Pear, Peel-of-mandarin-orange, Tomato, Green-pepper, Blackgram-Bean-sprouts, Eggplant, Lotus-root, Pumpkin, Shiitake, Ginger, Lettuce, Garlic, Japanese-honeywort, Aralia-cordata, Asparagus, Sasaveitchii, Clover, Kombu, Butterbur-sprout, Dandelion, Plantago-asiatica, Pea-bean-sprout, Cedar-leaf, Parsley, Turnip, Pineapple, Grape, Strawberry, Japanese-knotweed, Chives, Chinese-cabbage, Nettle-treemushroom, Salad, Chrysanthemum-coronarium, Artemisia-princeps, Oenanthe-javanica, Garlic-chives, Abies-sachalinensis, Shiso, Seaweed.

It is has been disclosed in Japanese Patent Laid Open No.4004-267100 that a fermented mugwort obtained by fermenting Artemisia-princeps, hence called as "mugwort", with Lactic-acid-bacteria improves an organoleptic and a flavor of food and drinks and is used for a blood pressure elevation-inhibiting agent and also is applicable for such food and drinks as Water-to-drink, Juice, Drink, Bread, Cake, Ricecracker, Baked-confectionery, Sweetened-and-jellied-bean-paste, Japanese-sweet, Ice-cream, Chewing-gum, Jelly, Bread, Confectionery, Udon, Soba, Noodles, Kamaboko, Ham, Fish-meat-sausage, Fish-meat-fish-paste, Miso, Soy-sauce, Dressing, Mayonnaise, Sweetener, Seasoning, Cheese, Butter, Yogurt, Ice-cream, Pudding, Dairy-products, Tofu, Konnyaku, Shellfish-boiled-in-sweetened-soy-sauce, Side-dish, Food and the like. Some improvements were found in the growth performance of chickens fed with Bacillus subtilis var. natto (Samanya M et al. Comparative Biochemistry and Physiology part A 133 (2002) 95-104) but the composition did not contain ginger or mugwort.

### DISCLOSURE OF INVENTION

### Technical Problem

As mentioned above, it is supposed that a herbal medicine and a spice which is effective for humans is also effective for livestock and poultry. However, a method to give ginger effectively to chicken is unknown. It is known that a fermented mugwort obtained by fermenting mugwort with Lactic-acid-bacteria is effective for food and drinks; foods. However, it is unknown to use the fermented mugwort obtained by fermenting mugwort with Lactic-acid-bacteria for gaining a body weight of chicken.

The inventor of the present invention has ended in failure in the experiment for giving ginger powder to chicken by adding it to a basal feed. However, the inventor of the present invention has known that a chicken eats ginger powder and grows well and the body weight of the chicken gains, even though a chicken-meat smells and tastes of ginger is not obtained, when the ginger powder is added to a basal feed together with the fermented mugwort obtained by fermenting mugwort with Lactic-acid-bacteria.

Further, the inventor of the present invention has known that not only a chicken but also a pig and sea-foods eat ginger powder and their body weight gain, when the ginger powder is added to a basal feed together with the fermented mugwort obtained by fermenting mugwort with Lactic-acid-bacteria.

Therefore, the objects of the present invention is to promote growth of animals, to gain body weight of animal, thereby to enhance a profit of animal raising industry by giving the animals the ginger powder by adding it to basal feeds together with the fermented mugwort as an animal body weight gain agent obtained by fermenting mugwort with Lactic-acid-bacteria.

### Means to solve technical problem

The first character of the present invention is a basal feed for chickens, containing an agent, which comprises a ginger powder and a fermented mugwort liquid having been obtained by fermenting mugwort with Bacillus-natto, in an amount of from 0.5 weight % to 1.5 weight % of the basal feed.

The second character of the present invention is a basal feed for fishes, containing an agent, which comprises a ginger powder and a fermented mugwort liquid having been obtained by fermenting mugwort with Bacillus-natto, in an amount of from 0.5 weight % to 1.5 weight % of the basal feed.

### Effect of invention

As mentioned above, chickens do not voluntarily eat the feed which the ginger is only added to. However, when the ginger is added to the basal feed together with the fermented mugwort obtained by fermenting mugwort with ferment bacteria, the chicken eats the feed which the ginger is added to. And it became unexpectedly apparent that the growth of chicken is promoted with the fermented mugwort ginger, the body weight of the chicken gains, and the death rate of young chickens decrease when the young chickens are raised with the assorted-feed consisting of a basal feed and 0.5 weight % to 2.0 weight % of the fermented mugwort ginger, that is, with the assorted-feed consisting of a basal feed containing vegetable oils and fats, animal oils and fats, saline and cereals which is amounted more than 50 % by weight of the basal feed and 0.5 weight % ∼ 2.0 weight % of the fermented mugwort ginger which is prepared by giving the ginger the fermented mugwort liquid which is obtained by fermenting the mugwort with at least a kind of ferment bacteria which is selected from the group consisting of Lactic-acid-bacteria, Yeast, Photosynthesis-bacteria, Actino-mycetes, Hyperthermal-bacteria, Aspergillus, Bacillus-subtilis and Bacillus-natto. These functions of the fermented mugwort ginger are also observed in raising of other animals, that is, in raising of a pig, a sea-food and the like. And, the growth of animals are promoted, the body weight of animals gain, the death rate of animals decrease, and then a profit of animal raising industry is enhanced by the application of the fermented mugwort ginger to the basal feeds.

### BRIEF DESCRIPTION OF DRAWINGS

Figure 1 shows a duplicate sectional plan view of intestine of yellowtail raised in accordance with the present invention.
Figure 2 shows a duplicate sectional plan view of intestine of yellowtail raised in accordance with prior art.

### Description of Notation

- 11 :: Intestine of yellowtail
- 12 :: Villi of intestine of yellowtail

### BEST MODE FOR CARRYING OUT THE INVENTION

The mugwort is ground into lanky powder and supplied into water together with such saccharoid as molasses, cane-sugar, glucose and the like. The ferment bacteria is added to the water and stood still for a few days. Then, the mugwort ferments. The fermented mugwort ginger is prepared by supplying the ginger to the fermented mugwort liquid prepared by fermenting the mugwort together with the ferment bacteria and the saccharoid in the water. At the time, it is encouraged to add further ferment bacteria and saline to the fermented mugwort liquid prepared before.

Lactic-acid-bacteria, Yeast, Photosynthesis-bacteria, Actino-mycetes, Hyperthermal-bacteria, Aspergillus, Bacillus-subtilis and Bacillus-natto may be applied as the ferment bacteria for fermenting the mugwort. It is preferable to apply Lactic-acid-bacteria as the ferment bacteria. It is more preferable to apply Lactic-acid-bacteria together with Photosynthesis-bacteria as the ferment bacteria.

In the case of application of the ferment bacteria to the fermented mugwort liquid prepared before, it is preferable to apply Bacillus-subtilis and Bacillus-natto to the additional ferment bacteria.

The amount about 1.0 weight %, that is, the amount 0.5 weight % ∼1.5 weight % of the fermented mugwort ginger is enough to add to the basal feed so that it is needless to add the basal feed more fermented mugwort ginger than 2.0 weight%.

A commercial feed for poultry and livestock containing cereals amounted more than 50 % by weight of the feed, vegetable oils and fats, animal oils and fats, saline, calcium-carbonate, calcium-phosphate and the like may be used for the basal feed for poultry and livestock.
A commercial feed for sea-foods mainly containing vegetable protein, animal protein, vegetable oils and fats, animal oils and fats, and additionally containing saline, calcium-carbonate, calcium-phosphate and the like may be used for the basal feed for sea-foods.

### EMBODIMENTS

### Embodiment 1

100 young chickens were divided into 4 groups of 1 st group, 2nd group, 3rd group and 4th group having 4 replicates of 25 birds in each group, were housed in at natural condition, that is, non regulations of temperature and humidity, and were raised for 7 weeks from June 12, 2007 till July 31, 2007.

The fermented mugwort ginger was not added to the basal feed given to the young chickens of the 1 st group.

0.5 weight % of the fermented mugwort ginger was added to the basal feed given to the young chickens of the 2nd group.

1.0 weight % of the fermented mugwort ginger was added to the basal feed given to the young chickens of the 3rd group.

2.0 weight % of the fermented mugwort ginger was added to the basal feed given to the young chickens of the 4th group.

The growth situations of young chickens of those 4 groups are shown with undermentioned [Table 1].

The fermented mugwort ginger was prepared by supplying a dry ginger powder to fermented mugwort liquid together with Bacillus-natto and by agitating them.

The fermented mugwort liquid was prepared by supplying finely ground mugwort to water containing molasses and Lactic-acid-bacteria.

For the first term from the 1st week till the 3rd week, the conventional feed, product name is "Power Chicken" of Nichiwasangyo Joint-stock company, containing 61 weight % of cereals (corn and milo), 29 weight % of vegetable oil lees (soybean oil lees and corn-gluten-meal), 7 weight % of animal feed (fish meals) and total 3 weight % of animal oils and fats, calcium-carbonate, saline and calcium-phosphate(alfalfa-meal) was used for the basal feed.

For the latter term from the 4th week till the 7th week, the conventional feed, product name is "Sukusuku Jump" of Nichiwasangyo Joint-stock company, containing 63 weight % of cereals (corn, milo and polished rice), 26 weight % of vegetable oil lees (soybean oil lees, rapeseed oil lees and sesame oil lees), 3 weight % of animal feed (chicken-meal: fish meals) and total 8 weight % of animal oils and fats, calcium-carbonate, saline and calcium-phosphate was used for the basal feed.

As shown with undermentioned [Table 1], the body weight of chicken of the 3rd group raised with the feed, which 1.0 weight % of the fermented mugwort ginger was added to, gained about 240 g compared with the body weight of chicken of the 1st group raised with the feed, which the fermented mugwort ginger was not added to, and the body weight of chicken of the 4th group raised with the feed which 2.0 weight % of the fermented mugwort ginger was added to.

All chickens of the 3rd group raised with the feed, which 1.0 weight % of the fermented mugwort ginger was added to, had not died.

In this manner, it was confirmed that the good result was obtained in the case of the amount of addition of fermented mugwort ginger added to the basal feed was designed about 1.0 weight %.

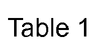

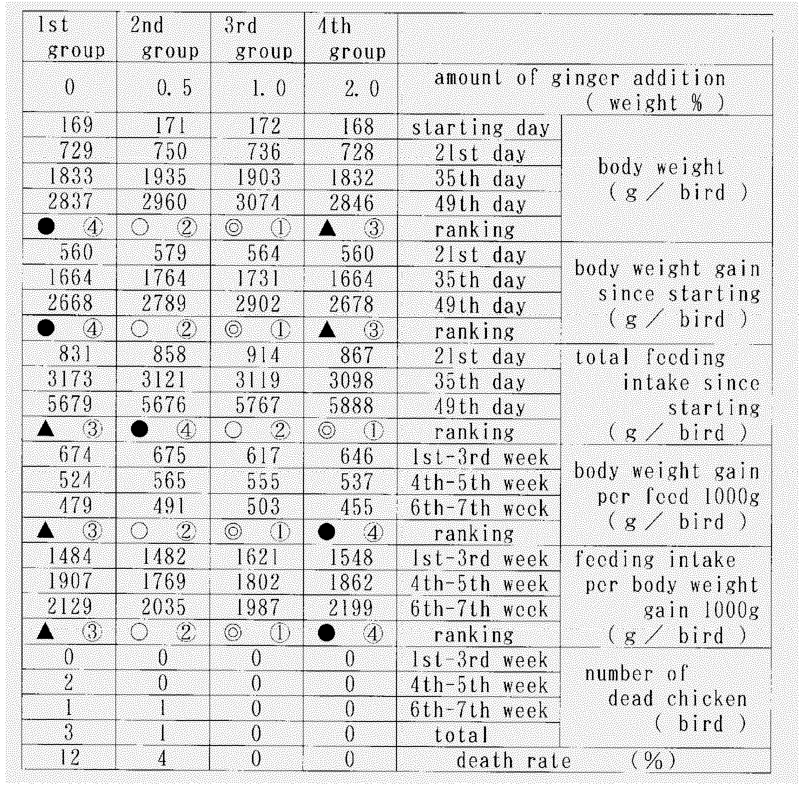

For reconfirmation of abovementioned result, intestinal epithelial cells of chickens of each groups of 1st group, 2nd group, 3rd group and 4^{th} group were investigated. And, in comparison with the chickens of the 1st group raised without fermented mugwort ginger, increasing tendencies of villus height and epithelial cell area of intestine were confirmed in the chickens of 2nd group, 3rd group and 4th group raised with fermented mugwort ginger. Further, among the chickens of 2nd group,3rd group and 4th group raised with fermented mugwort ginger, tendencies of increasing of epithelial cell area of intestine, projection of protuberated epithelial cell, and elongation of filament liked bacteria floating out from epithelial cell in proportion to increasing of the amount of addition of fermented mugwort ginger were confirmed. On the other hand, the epithelium cell of apical surface of villus of epithelial cell of intestine of the chickens of the 1st group raised without fermented mugwort ginger had an appearance as if fine villi were roughly arranged side by side. As mentioned above, the functions and utilities of the fermented mugwort ginger to hypertrophy the epithelial cell of chicken, to enhance the activity of intestine of chicken, to increase the chicken-raising efficacy of feed, to gain body weight of chicken, and to enhance the chicken-raising productivity were confirmed.

### Embodiment 2

16 young chickens were divided into 2 groups of 1st group and 2nd group having 2 replicates of 8 birds in each group, were housed in at natural condition, that is, non regulations of temperature and humidity, and were raised for 7 weeks from August 3, 2007 till September 21, 2007.

The fermented mugwort ginger was not added to the basal feed given to the young chickens of the 1 st group.

1.0 weight % of the fermented mugwort ginger was added to the basal feed given to the young chickens of the 2nd group.

The growth situations of young chickens of those 2 groups are shown with undermentioned [Table 2].

For the first term from the 1st week till the 3rd week, the conventional feed, product name is "Power Chicken" of Nichiwasangyo Joint-stock company, used in the abovementioned Embodiment 1 was used for the basal feed.

For the latter term from the 4th week till the 7th week, the conventional feed, product name is "Sukusuku Jump" of Nichiwasangyo Joint-stock company, used in the abovementioned Embodiment 1 was used for the basal feed.

As shown with undermentioned [Table 2], the body weight of chicken of the 2nd group raised with the feed, which 1.0 weight % of the fermented mugwort ginger was added to, gained about 200 g compared with the body weight of chicken of the 1st group raised without fermented mugwort ginger. In this manner, it was reconfirmed that the good result was obtained in the case of the amount of addition of fermented mugwort ginger added to the basal feed was designed about 1.0 weight %.

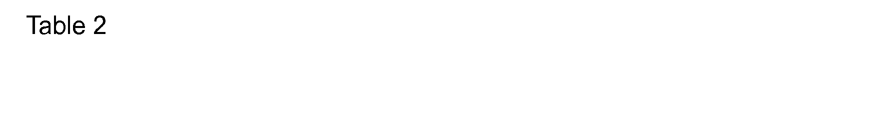

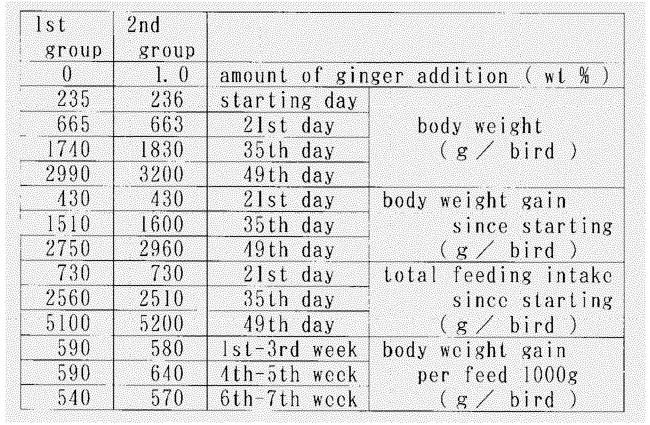

### Embodiment 3

40 young yellowtails were divided into 4 groups of 1 st group, 2nd group, 3rd group and 4th group having 4 replicates of 10 fish in each group, were put into the fish tanks at natural condition, that is, non regulations of temperature, and were raised for 30 days from November 5, 2007 till December 5, 2007.

The fermented mugwort ginger was not added to the basal feed given to the young yellowtails of the 1 st group and the 2nd group.

1.0 weight % of the fermented mugwort ginger was added to the basal feed given to the young yellowtails of the 3rd group and the 4th group. The growth situations of young yellowtails of those 4 groups are shown with undermentioned [Table 3].

The fermented mugwort ginger was prepared by supplying a dry ginger powder to fermented mugwort liquid together with Bacillus-natto and by agitating them. The fermented mugwort liquid was prepared by supplying finely ground mugwort to water containing saline, milk, molasses and Lactic-acid-bacteria.

The non-assorted-feed not containing the fermented mugwort ginger was prepared by mixing the conventional feed for yellowtail named "Buriyoumasshu" which was product name of Nisshinmarubeni-feed Joint-stock company, the conventional fish oils named "Feed Oil Ω" which was product name of Riken Joint-stock company and cellulose of Wakojunyaku Joint-stock company make, and by further mixing and adding 450 weight % of water.

The assorted-feed containing the fermented mugwort ginger was prepared by mixing the conventional feed for yellowtail named "Buriyoumasshu" which was product name of Nisshinmarubeni-feed Joint-stock company, the conventional fish oils named "Feed Oil Ω" which was product name of Riken Joint-stock company and cellulose of Wakojunyaku Joint-stock company make, by adding 1.0 weight % of the fermented mugwort ginger, and by further mixing and adding 450 weight % of water.

In the undermentioned [Table 3], the body weight gain efficiency (E) of young yellowtail during the raising period was calculated as follows;

1st step; the difference (D) between total weight (X) of 10 yellowtails in each fish tank on the raise starting day and total weight (Y) of 10 yellowtails in each fish tank on the measuring body weight day is calculated.
2nd step; the divided value (Z) is calculated by dividing the difference (D) with total weight (X) of 10 yellowtails in each fish tank on the raise starting day.
3rd step; the body weight gain efficiency (E) is calculated by multiplying the divided value (Z) by 100.

The feeding efficiency (F) during the raising period was calculated as follows;
1st step; the difference (D) between total weight (X) of 10 yellowtails in each fish tank on the raise starting day and total weight (Y) of 10 yellowtails in each fish tank on the measuring body weight day is calculated.
2nd step; the divided value (W) is calculated by dividing the difference (D) with total feeding intake (V) given to 10 yellowtails during the body weight measuring period.
3rd step; the feeding efficiency (F) is calculated by multiplying the divided value (W) by 100.

As the data of 15th day after raise starting in the undermentioned [Table 3] shown, the body weight gain efficiency (E) of yellowtail of 3rd and 4th groups raised with the assorted-feed containing the fermented mugwort ginger are about 2.5 weight % more than the body weight gain efficiency (E') of yellowtail of 1 st and 2nd groups raised with the non-assorted-feed not containing the fermented mugwort ginger.
As the data of 30th day after raise starting in the undermentioned [Table 3] shown, the body weight gain efficiency (E) of yellowtail of 3rd and 4th groups raised with the assorted-feed containing the fermented mugwort ginger are about 3.1 weight % more than the body weight gain efficiency (E') of yellowtail of 1 st and 2nd groups raised with the non-assorted-feed not containing the fermented mugwort ginger.
In this manner, the effect of weight gain caused by the fermented mugwort ginger was confirmed.

As the data of 15th day after raise starting in the undermentioned [Table 3] shown, the feeding efficiency (F) of yellowtail of 3rd and 4th groups raised with the assorted-feed containing the fermented mugwort ginger are about 2.5 weight % more than the feeding efficiency (F') of yellowtail of 1st and 2nd groups raised with the non-assorted-feed not containing the fermented mugwort ginger.
As the data of 30th day after raise starting in the undermentioned [Table 3] shown, the feeding efficiency (F) of yellowtail of 3rd and 4th groups raised with the assorted-feed containing the fermented mugwort ginger are about 5.9 weight % more than the feeding efficiency (F') of yellowtail of 1st and 2nd groups raised with the non-assorted-feed not containing the fermented mugwort ginger.
In this manner, the effect of feeding caused by the fermented mugwort ginger was confirmed.

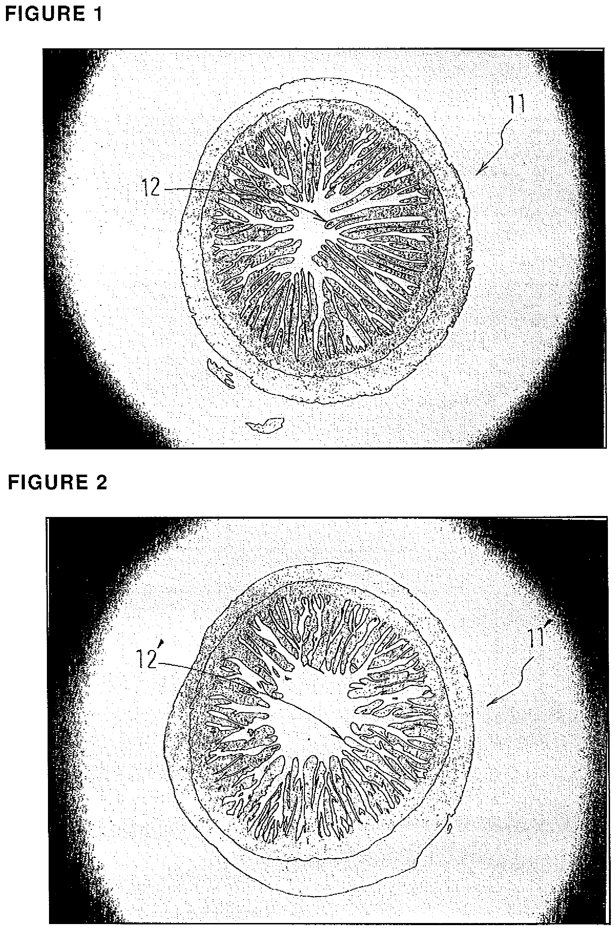

The section of the epithelial cells of intestine (11) of the yellowtail of 30th day after raise starting raised with the assorted-feed containing the fermented mugwort ginger was taken on electron microscope and investigated (Figure 1).
The section of the epithelial cells of intestine (11') of the yellowtail of 30th day after raise starting raised with the non-assorted-feed not containing the fermented mugwort ginger was also taken on electron microscope and investigated (Figure 2).
The villi (12) of intestine (11) of the yellowtail raised with the assorted-feed containing the fermented mugwort ginger (Figure 1) were finer and more compact than the villi (12') of intestine (11') of the yellowtail raised with the non-assorted-feed not containing the fermented mugwort ginger (Figure 2).
In this manner, the effect of feeding caused by the fermented mugwort ginger was confirmed with the electron microscopes.

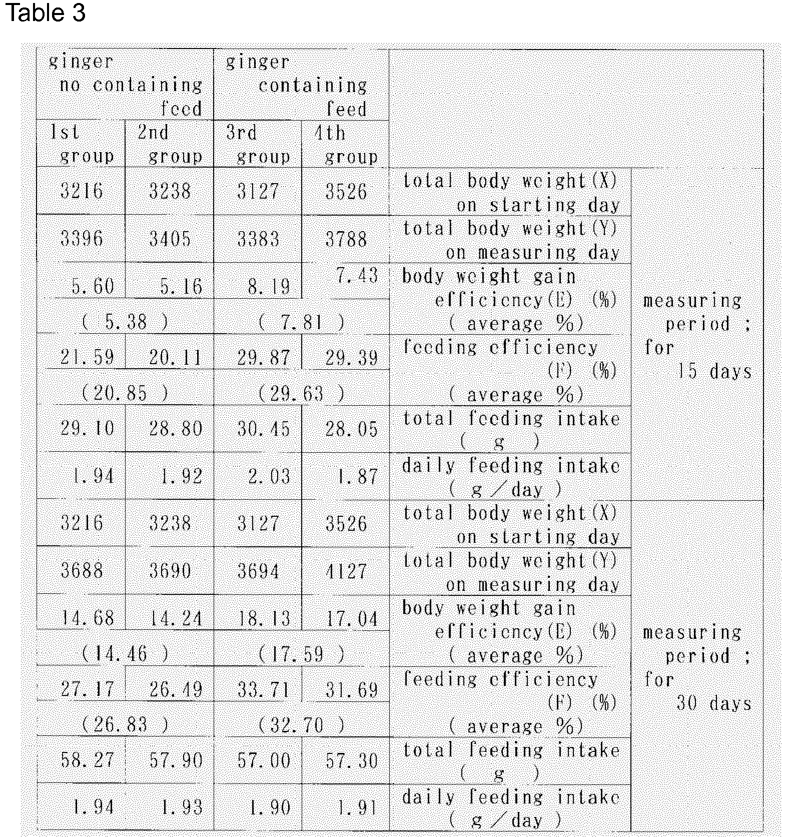

### INDUSTRIAL APPLICABILITY

As mentioned above, the present invention is useful to promote growth of animals and humans, to gain body weight of animals and humans, to enhance a profit of animal raising industry and to make human healthy.

## Claims

1. A basal feed for chickens, containing an agent, which comprises a ginger powder and a fermented mugwort liquid having been obtained by fermenting mugwort with Bacillus-natto, in an amount of form 0.5 weight % to 1.5 weight % of the basal feed.

2. A basal feed for fishes, containing an agent, which comprises a ginger powder and a fermented mugwort liquid having been obtained by fermenting mugwort with Bacillus-natto, in an amount of form 0.5 weight % to 1.5 weight % of the basal.

## Patentansprüche

1. Ein Basal Futter für Hühner, enthaltend ein Arzneimittel, das ein Ingwer Pulver und eine fermentierte Beifuß Flüssigkeit enthält, die durch Fermentieren von Beifuß mit Bacillus-natto in einer Menge von 0,5 Gewicht % bis 1,5 Gewicht % des Basal Futter erhalten worden.

2. Ein Futter für Fische, enthaltend ein Arzneimittel, das ein Ingwer Pulver und eine fermentierte Beifuß Flüssigkeit enthält, die durch Fermentieren von Beifuß mit Bacillus-natto in einer Menge von 0,5 Gewicht % bis 1,5 Gewicht % des Basal Futter erhalten worden.

## Revendications

1. Une alimentation basale pour des poulets, contenant un agent, qui comprend une poudre de gingembre et un liquide de armoise ferment ayant été obtenu par fermentent les armoise avec Bacillus-natto, en quantité de 0,5% en poids à 1,5% en poids de les alimentation basale.

2. Une alimentation basale pour des poissons, contenant un agent, qui comprend une poudre de gingembre et un liquide de armoise ferment ayant été obtenu par fermentent les armoise avec Bacillus-natto, en quantité de 0,5% en poids à 1,5% en poids de les alimentation basale.
